# EUROPEAN PATENT APPLICATION

(11) **EP 1 972 337 A2**
(43) Date of publication of application: **24.09.2008**
(21) Application number: 08102385.5
(22) Date of filing: 07.03.2008
(51) Int. Cl.: A61K 9/16, A61K 9/20

(54) **Excipient and an improved method for manufacturing extracted, evaporated, granulated botanical herb product**

(30) Priority: 21.03.2007 TW 96109647
(71) Applicant: Kaiser Pharmaceutical Co., Ltd., 9, Hwan Kung Road Yungkang, 71041 Tainan (TW)
(72) Inventor: TSsai, Sung-I, 71041 Tainan (TW)
(74) Representative: 2K Patentanwälte Kewitz & Kollegen

(57) **Abstract**

An excipient and a method for manufacturing concentrated Chinese herbs through using the excipient are provided. The manufacturing method includes the following steps: (a) providing a Chinese herb raw material; (b) obtaining an extract from the Chinese herb raw material; (c) collecting volatile oils in a separate bin, later reintroduced; (d) removing water from the extract, to get a concentrate; and (e) adding an excipient to the concentrate, and then granulating and drying, to get a Chinese herbal preparation, in which the excipient contains a capsule protective agent of 1-99 wt% with respect to the excipient and a decomposed product of starch of 1-99 wt% with respect to the excipient. Therefore, the amount of starch required to be used is reduced and the manufactured concentrated Chinese herbal preparation can be completely dissolved in water.

## Description

### BACKGROUND OF THE INVENTION

### 1. Field of the Invention

The present invention relates to the manufacturing of extracted, evaporated, granulated, botanical herb product, usually TCM (traditional Chinese medicine) herbs using a specific procedure and excipient method. More particularly, the present invention relates to an excipient comprising a capsule protective agent and a decomposed product of starch, and a method for manufacturing concentrated Chinese herbs using the excipient.

### 2. Description of the Related Art

In recent years, with the progress of biochemical science and technology, traditional Chinese herb medicine has been more and more widely applied. Unlike dose forms of the traditional Chinese herb medicine, i.e., decoction, pill, powder, paste, pellet, wine, distillate, and tablet, dose forms of modem Chinese herb medicine include powder, granule, capsule, tablet, solution, tape, and so on. With the development of science, Chinese herbal medicine manufacturers in Taiwan have already followed GMP regulations in order to obtain high-quality products.

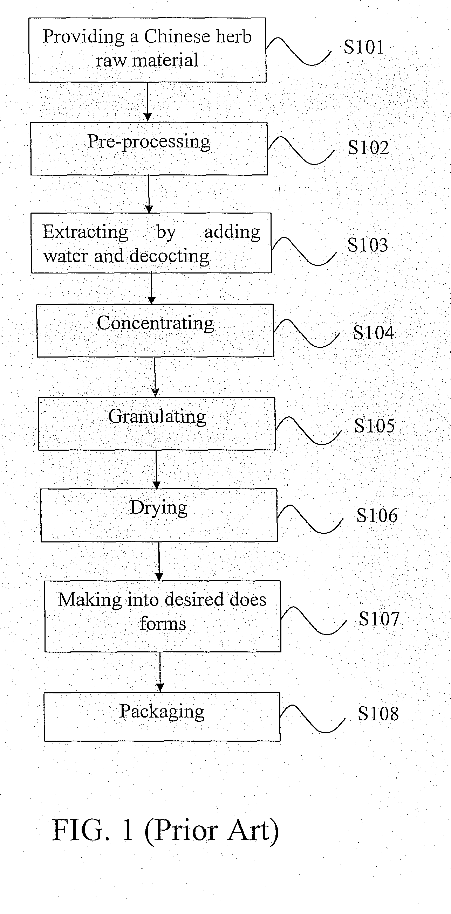

FIG. 1 shows a schematic flow chart of a conventional method for manufacturing concentrated Chinese herbs. First, in Step S101, a Chinese herb raw material is provided. "Chinese herb raw material" herein refers to a product name or prescription according to original works such as *Golden Mirror of Medicine, Collected Explanations of Medical Formulas, Compendium of Materia Medica, Encyclopaedic Dictionary of Chinese Medicine,* or *Prescriptions of the Bureau of Taiping People's Welfare Pharmacy* which is generally an accepted Chinese herbal preparation.

Next, in Step S102, the Chinese herb raw material is pre-processed, generally including assessing the appearance, nature, feature and taste, testing the herb's ingredients, cleaning, and formulating, and so on.

Then, in Step S103, the Chinese raw herb is extracted using a solvent and decoction process. In this step, usually large amount of water or a mixture of water and ethanol is boiled with the Chinese herb raw material at a temperature of about 100°C. The resulting extract contains a variety of marker or active ingredients from the Chinese herb raw material, for example, saccharides, glycosides, organic acids, phenols, and alkaloids.

Next, Step S104 is a concentration step, including heating the extract obtained in Step S103 to evaporate the moisture and to increase the concentration, so as to get a concentrate.

Then, Step S105 is a granulation step, including placing the concentrate and an excipient into a mixing granulator and mixing them for a proper period of time to form particles. Generally, the excipient is starch.

Next, Step S106 is a drying step, including drying the particles in Step S105 for a proper period of time.

Then, in Step S107, the dried particles are made into desired dose forms, such as powder, particle, capsule, or tablet. Finally, Step S108 is a packaging step, including packaging the preparations in desirable dose forms in Step S107 into final products for sale.

The disadvantages of the conventional manufacture method lie in the fact that the excipient added in Step S105 is merely starch, and generally, a large amount of excipient is required to get desirable molding effects. However, adding too much starch may affect the absorption and metabolism of the active ingredients in the Chinese herbs and may cause the final product to be incompletely dissolved in water, and thus generate precipitates, resulting in a poor taste. Further, volatile oils are lost during decoction with the conventional method.

As disclosed in Taiwan Patent Publication No. 457091, entitled "Concentrated Chinese Herbal Preparation and Method for Making the Same," a surfactant, such as sucrose fatty acid esters, sorbitan fatty acid esters, glycerin fatty acid esters, or a mixture thereof is used as an excipient, but the effects are still not significantly improved.

Therefore, it is necessary to provide an excipient and a method for manufacturing concentrated Chinese herbs using the excipient to solve the above problems. In addition, methods for volatile oils retention, supercritical extraction, and spray dryer or flow coating granulation process are applied in the present invention for an enhanced final botanical product.

### SUMMARY OF THE INVENTION

The present invention is directed to a method for manufacturing concentrated Chinese herbs, which includes the following steps: (a) providing a Chinese herb raw material; (b) obtaining an extract from the Chinese herb raw material; (c) removing water from the extract, to get a concentrate; and (d) adding an excipient to the concentrate, and then granulating and drying, to get a concentrated Chinese herbal preparation, in which the excipient contains a capsule protective agent of 1-99 wt% with respect to the excipient and a decomposed product of starch of 1-99 wt% with respect to the excipient. Using the excipient formula of the present invention, the resulting final product has a reduced excipient content, and greater water solubility. Additionally, after being dissolved in water, the taste of the product of the present invention is similar to that obtained by decoction, as if water reconstituted a fresh brew. Furthermore, the present invention has the advantages of improved activity, being safe and effective, being easily absorbed, and being capable of being completely dissolved without precipitates. The product of the present invention is a new ingredient having new uses (to be brewed for drinking).

The present invention is further directed to an excipient used in a method for manufacturing concentrated Chinese herbs which contains a capsule protective agent of 1-99 wt% with respect to the excipient and a decomposed product of starch of 1-99 wt% with respect to the excipient.

### BRIEF DESCRIPTION OF THE DRAWINGS

FIG. 1 is a schematic flow chart of a conventional method for manufacturing concentrated Chinese herbs;
FIG. 2 is a schematic flow chart of a method for manufacturing concentrated Chinese herbs according to a first embodiment of the present invention; and
FIG. 3 is a schematic flow chart of a method for manufacturing concentrated Chinese herbs according to a second embodiment of the present invention.

### DETAILED DESCRIPTION OF THE INVENTION

"Chinese herb raw material" herein refers to a product name or prescription according to original works such as *Golden Mirror of Medicine, Collected Explanations of Medical Formulas, Compendium of Materia Medica, Encyclopaedic Dictionary of Chinese Medicine,* or *Prescriptions of the Bureau of Taiping People's Welfare Pharmacy* which is generally an accepted Chinese herbal preparation.

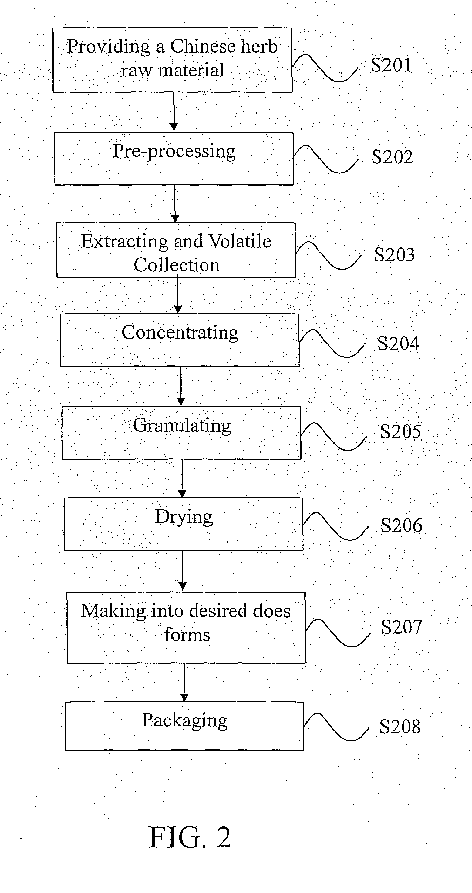

FIG. 2 shows a schematic flow chart of a method for manufacturing concentrated Chinese herbs according to a first embodiment of the present invention. First, in Step S201, a Chinese herb raw material is provided. Next, in Step S202, the Chinese herb raw material is pre-processed, which generally includes assessing the appearance, nature, feature and taste, testing the herb ingredients, cleaning, and formulating, and so on. The pre-processing step preferably further includes crumbing and mixing the Chinese herb raw material using a homogenizer, and taking the Chinese herb raw material of 4-200 mesh.

Thereafter, in Step S203, an extract is obtained from the Chinese herb raw material. An extract may be obtained from the Chinese herb raw material by using supercritical carbon dioxide or under the pressure of higher than 1 atm, or conventionally by decocting with water medium or a mixture of water and ethanol. The extract comprises a variety of marker or active ingredients of the Chinese herb raw material, for example, saccharides, glycosides, and organic acids. In a preferred example, during this decoction process, the volatile oils are evaporated and collected in a separate bin, cooled, stored until later reintroduction.

Then, a concentration step, Step S204, requires heating and/or filtering the extract obtained in Step S203 to evaporate/separate the solvent, as to get a concentrated extract (called "immercing paste"). The extract obtained in Step S203 is preferably first centrifugally filtered, and then concentrated in a pressure-reduction concentrator at a low temperature (lower than 60°C).

Next, a granulation step, Step S205, requires mixing the concentrate obtained in Step S204 with stored volatile oils obtained in Step S203 and an excipient for a certain period of time, and then gradually spraying it into a spray dryer by means of a vacuum or hot wind to form particles. In this embodiment, the excipient comprises a capsule protective agent of 1-99 wt% with respect to the excipient and a decomposed product of starch of 1-99 wt% with respect to the excipient. The added amount of the excipient is a ratio with respect to a solid in the concentrate. For example, if the solid in the concentrate is 1-15 wt%, the added amount of the excipient must make the capsule protective agent be about 1-15 wt% with respect to the solid; if the solid in the concentrate is 16-29 wt%, the added amount of the excipient must make the capsule protective agent be about 16-29 wt% with respect to the solid; if the solid in the concentrate is 30-39 wt%, the added amount of the excipient must make the capsule protective agent be about 30-39 wt% with respect to the solid; if the solid in the concentrate is 40-49 wt%, the added amount of the excipient must make the capsule protective agent be about 40-49 wt% with respect to the solid.

The capsule protective agent is selected from a group consisting of gelatinized starch (alkaline-treated starch), gellan gum, curdlan, alginic acid, acacia, hydroxypropyl methylcellulose (propylene glycol ether of methycellulose), hydroxypropyl cellulose, carrageenan, xanthan gum, maltodextrin, cyclodextrin, and a mixture thereof.

The decomposed product of starch is selected from a group consisting of maltodextrin, cyclodextrin; and a mixture thereof. The added amount of the decomposed product of starch is not particularly limited.

Thereafter, Step S206 is a drying step, drying the particles in Step S205 for a proper period of time. The dried particles may preferably be meshed by a screen.

Then, in Step S207, the dried particles are made into desired dose forms, for example, powder, granules, capsules, or tablets. Finally, Step S208 is a packaging step, including packaging preparations in desired dose forms in Step S207 in final products for sale.

The present invention has the advantages that the used excipient includes a capsule protective agent and a decomposed product of starch, thus reducing the amount of the required starch and having better effects than those obtained merely with starch added. Additionally, after being dissolved in water, the taste of the product of the present invention is similar to that obtained by decoction, based on reducing and quenching processes. Furthermore, the present invention has the advantages of improved activity, being safe and effective, being easily absorbed, and being capable of being completely dissolved without precipitates. The product of the present invention is a new ingredient having new uses (to be brewed for drinking).

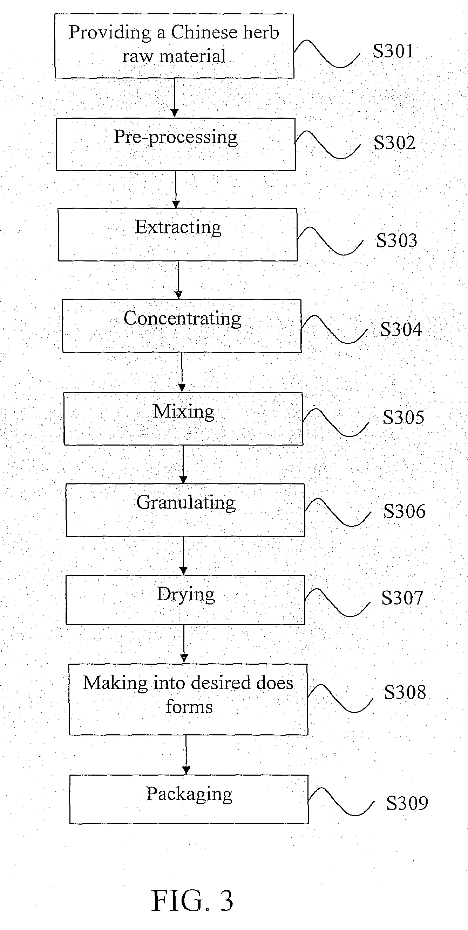

FIG. 3 shows a schematic flow chart of a method for manufacturing concentrated Chinese herbs according to a second embodiment of the present invention. First, in Step S301, a Chinese herb raw material is provided. Next, in Step S302, the Chinese herb raw material is pre-processed, which generally includes assessing the appearance, nature, feature and taste, testing the herb ingredients, cleaning, and formulating, and so on.

Thereafter, in Step S303, an extract is obtained from the Chinese herb raw material. An extract may be obtained from the Chinese herb raw material by using supercritical carbon dioxide or under the pressure of higher than 1 atm, or conventionally by decocting with water medium or a mixture of water and ethanol. The extract comprises a variety of marker or active ingredients of the Chinese herb raw material, for example, saccharides, glycosides, and organic acids. In a preferred example, during this decoction process, the volatile oils are evaporated and collected in a separate bin, cooled, stored until later reintroduction.

Then, a concentration step, Step 304, requires heating and/or filtering the extract obtained in Step S303 to evaporate/separate the solvent, as to get a concentrated extract (called "immercing paste"). The extract obtained in Step S303 is preferably first centrifugally filtered, and then concentrated in a pressure-reduction concentrator at a low temperature (lower than 60°C).

Next, Step S305 is a mixing step, including thoroughly mixing the concentrate obtained in Step S304 with a first capsule protective agent. The added amount of the first capsule protective agent is a ratio with respect to a solid in the concentrate. For example, if the solid in the concentrate is 1-15 wt%, the added amount of the first capsule protective agent is about 1-15 wt% with respect to the solid; if the solid in the concentrate is 16-29 wt%, the added amount of the first capsule protective agent is about 16-29 wt% with respect to the solid; if the solid in the concentrate is 30-39 wt%, the added amount of the first capsule protective agent is about 30-39 wt% with respect to the solid; and if the solid in the concentrate is 40-49 wt%, the added amount of the first capsule protective agent is about 40-49 wt% with respect to the solid.

The first capsule protective agent is selected from a group consisting of gelatinized starch (alkaline-treated starch), gellan gum, curdlan, alginic acid, acacia, hydroxypropyl methylcellulose (propylene glycol ether of methycellulose), hydroxypropyl cellulose, carrageenan, xanthan gum, maltodextrin, cyclodextrin, and a mixture thereof.

Then, Step S306 is a granulation step, including placing an excipient into a spray drier, or individually placing the decomposed product of starch or starch into a spray drier. In this embodiment, the excipient contains a second capsule protective agent of 1-99 wt% with respect to the excipient and a decomposed product of starch of 1-99 wt% with respect to the excipient. The added amounts of the excipient and the second capsule protective agent are not particularly limited.

The second capsule protective agent is selected from a group consisting of gelatinized starch (alkaline-treated starch), gellan gum, curdlan, alginic acid, acacia, hydroxypropyl methylcellulose (propylene glycol ether of methycellulose), hydroxypropyl cellulose, carrageenan, xanthan gum, maltodextrin (TR), and a mixture thereof. It should be noted that the second capsule protective agent can be identical with or different from the first capsule protective agent. The decomposed product of starch is selected from a group consisting of maltodextrin, cyclodextrin, and a mixture thereof. The added amount of the decomposed product of starch is not particularly limited.

Thereafter, the concentrate obtained in Step S305 is gradually sprayed into the spray drier, so as to form particles. Afterwards, in a preferred example, the volatile oils collected in step S303 are added.

Next, Step S307 is a drying step, including drying the particles in Step S306 for a proper period of time. The dried particles are preferably further screened by a screen.

Then, in Step S308, the dried particles are made into desired dose forms, for example, powder, granule, capsule, or tablet. Finally, Step S309 is a packaging step, including packaging the preparations with desired dose forms in Step S308 into final products for sale.

The present invention will be described in detail below with reference to the embodiments, which are not intended to limit the present invention to the disclosure of these embodiments.

### Example 1: Method for Manufacturing Hsiang Sheng Po Ti Wan

The components of the Chinese herb raw material in this embodiment are listed as follows:

| | |
|---|---|
| Peppermint | 1800 g |
| Chuanxiong Rhizome | 1200 g |
| Forsythia Fruit | 1200 g |
| Glycyrrhiza | 900 g |
| Common Cephalanoplos Herb | 900 g |
| Platycodon Root | 900 g |
| Rhubarb | 450 g |
| Villous Amomum Fruit | 450 g |
| Medicine Terminalia Fruit | 450 g |

The steps of the manufacturing method are listed as follows.
1. 82.5 L to 250 L of pure water was added to 8.25 kg of the pre-processed Chinese herb raw material and then heated to boiling for several hours, and then centrifugally filtered, to get an extract. During this extraction process, the volatile oils were evaporated, condensed, and collected at a separate bin.
2. The extract of Step 1 was concentrated in a pressure-reduction concentrator at a temperature lower than 60°C, to get a concentrate of about 5 kg.
3. About 5 kg of the concentrate obtained in Step 2 was thoroughly mixed with an excipient, which contained 270 g of a capsule protective agent and 200 g of a decomposed product of starch. As the concentrate was about 5 kg, and the solid therein was about 20 wt%, the added amount of the capsule protective agent was 5000*20%*27%=270 g. The capsule protective agent includes gelatinized starch, gellan gum, acacia, and carrageenan. The decomposed product of starch is maltodextrin, and the added amount thereof is not particularly limited, but can be adjusted according to the concentration ratio or taste.
4. The concentrate obtained in Step 3 was gradually sprayed into a dryer by means of a vacuum or hot wind to form particles, and then dried for 30 min after spraying. Afterwards, the volatile oils were added.
5. The dried particles were screened by screens of 20 mesh and 100 mesh to obtain the particles of 20-100 mesh.
6. The particle product obtained in Step 5 was made into a tablet product through a tablet machine.
7. The tablet product obtained in Step 6 was packaged.

### Example 2: Method for Manufacturing Hsiang Sheng Po Ti Wan

The components of the Chinese herb raw material in this embodiment are listed as follows:

| | |
|---|---|
| Peppennint | 1800 g |
| Chuanxiong Rhizome | 1200 g |
| Forsythia Fruit | 1200 g |
| Glycyrrhiza | 900 g |
| Common Cephalanoplos Herb | 900 g |
| Platycodon Root | 900 g |
| Rhubarb | 450 g |
| Villous Amomum Fruit | 450 g |
| Medicine Terminalia Fruit | 450 g |

The steps of the manufacturing method are listed as follows.
1. 82.5 L to 250 L of pure water was added to 8.25 kg of the pre-processed Chinese herb raw material and then heated to boiling for several hours, and then centrifugally filtered, to get an extract. During this extraction process, the volatile oils were evaporated, condensed, and collected at a separate bin.
2. The extract in Step 1 was concentrated in a pressure-reduction concentrator at a temperature lower than 60°C, to get a concentrate of about 5 kg.
3. About 5 kg of the concentrate in Step 2 was thoroughly mixed with a first capsule protective agent of 270 g. As the concentrate was about 5 kg, and the solid therein was about 20 wt%, the added amount of the first capsule protective agent was 5000*20%*27%=270 g. The first capsule protective agent includes gelatinized starch, gellan gum, acacia, and carrageenan.
4. An excipient of 2500 g containing a second capsule protective agent of 1250 g and a decomposed product of starch of 1250 g was placed into a spray drier. The added amount of the second capsule protective agent is about one fourth of that of the concentrate. The second capsule protective agent includes gelatinized starch, gellan gum, acacia, and carrageenan. The decomposed product of starch is maltodextrin, and the added amount thereof is not particularly limited, but can be adjusted according to the concentration ratio or taste.
5. The concentrate obtained in Step 3 was gradually sprayed into the spray dryer to form particles, and then dried for 30 min after spraying. Afterwards, the volatile oils were added.
6. The dried particles were screened by screens of 20 mesh and 100 mesh to obtain the particles of 20-100 mesh.
7. The particle product obtained in Step 6 was made into a tablet product through a tablet machine.
8. The tablet product obtained in Step 7 was packaged.

### Example 3: Method for Manufacturing Chuan Chiong Char Tiao San

The components of the Chinese herb raw material in this embodiment are listed as follows:

| | |
|---|---|
| Peppermint | 1600 g |
| Chuanxiong Rhizome | 800 g |
| Schizonepeta Herb | 800 g |
| Dahurian Angelica Root | 400 g |
| Glycyrrhiza | 400 g |
| Notopteryygium Rhizome | 400 g |
| Rhubarb | 450 g |
| Saposhnikovia Root | 300 g |
| Asarum Herb | 200 g |

The steps of the manufacturing method are listed as follows.
1. 49 L to 145 L of pure water was added to 4.9 kg of the pre-processed Chinese herb raw material and then heated to boiling for several hours, and then centrifugally filtered, to get an extract.
2. The extract in Step 1 was concentrated in a pressure-reduction concentrator at a temperature lower than 60°C, to get a concentrate of about 2.5 kg.
3. About 2.5 kg of the concentrate obtained in Step 2 was thoroughly mixed with an excipient, which contained a capsule protective agent of 135 g and a decomposed product of starch of 90 g. As the concentrate was about 2.5 kg, and the solid therein was about 20 wt%, the added amount of the capsule protective agent was 2500*20%*27%=135 g. The capsule protective agent includes gelatinized starch, gellan gum, acacia, and carrageenan. The decomposed product of starch is maltodextrin, and the added amount thereof is not particularly limited, but can be adjusted according to the concentration ratio or taste.
4. The concentrate obtained in Step 3 was gradually sprayed into a dryer by means of a vacuum or hot wind to form particles, and then dried for 30 min after spraying.
5. The dried particles were screened by screens of 20 mesh and 100 mesh to obtain the particles of 20-100 mesh.
6. The particle product obtained in Step 5 was made into a tablet product through a tablet machine.
7. The tablet product obtained in Step 6 was packaged.

### Example 4: Method for Manufacturing Chuan Chiong Char Tiao San

The components of the Chinese herb raw material in this embodiment are listed as follows:

| | |
|---|---|
| Peppermint | 1600 g |
| Chuanxiong Rhizome | 800 g |
| Schizonepeta Herb | 800 g |
| Dahurian Angelica Root | 400 g |
| Glycyrrhiza | 400 g |
| Notopteryygium Rhizome | 400 g |
| Rhubarb | 450 g |
| Saposhnikovia Root | 300 g |
| Asarum Herb | 200 g |

The steps in the manufacturing method are listed as follows:
1. 49 L to 145 L of pure water was added to 4.9 kg of the pre-processed Chinese herb raw material and then heated to boiling for several hours, and then centrifugally filtered, to get an extract.
2. The extract in Step 1 was concentrated in a pressure-reduction concentrator at a temperature lower than 60°C, to get a concentrate of about 2.5 kg.
3. About 2.5 kg of the concentrate of Step 2 was thoroughly mixed with a first capsule protective agent of 135 g. As the concentrate was about 2.5 kg, and the solid therein was about 20 wt%, the added amount of the first capsule protective agent was 2500*20%*27%=135 g. The first capsule protective agent includes gelatinized starch, gellan gum, acacia, and carrageenan.
4. An excipient of 1250 g containing a second capsule protective agent of 625 g and a decomposed product of starch of 625 g was placed into a spray drier. The added amount of the second capsule protective agent is about one fourth of that of the concentrate. The second capsule protective agent includes gelatinized starch, gellan gum, acacia, and carrageenan. The decomposed product of starch is maltodextrin, and the added amount thereof is not particularly limited, but can be adjusted according to the concentration ratio or taste.
5. The concentrate obtained in Step 3 was gradually sprayed into the spray dryer to form particles, and then dried for 30 min after spraying.
6. The dried particles were screened by screens of 20 mesh and 100 mesh to obtain the particles of 20-100 mesh.
7. The particle product obtained in Step 6 was made into a tablet product through a tablet machine.
8. The tablet product obtained in Step 7 was packaged.

While several embodiments of the present invention have been illustrated and described, various modifications and improvements can be made by those skilled in the art. The embodiments of the present invention are therefore described in an illustrative but not restrictive sense. It is intended that the present invention should not be limited to the particular forms as illustrated, and that all modifications which maintain the spirit and scope of the present invention are within the scope defined in the appended claims.

## Claims

1. A method for manufacturing extracted, evaporated, granulated botanical herb products such as concentrated Chinese herbs, comprising:
(a) providing a Chinese herb raw material;
(b) obtaining an extract from the Chinese herb raw material;
(c) removing water from the extract, to get a concentrate; and
(d) adding an excipient to the concentrate, and then granulating and drying, to get a concentrated Chinese herbal preparation, wherein the excipient contains a capsule protective agent of 1-99 wt% with respect to the excipient and a decomposed product of starch of 1-99 wt% with respect to the excipient.

2. The method as claimed in Claim 1, further comprising a step of pre-processing the Chinese herb raw material after Step (a).

3. The method as claimed in Claim 1 or 2, wherein Step (b) comprises extracting the Chinese herb raw material by supercritical carbon dioxide.

4. The method as claimed in Claim 3, wherein Step (b) is performed at a pressure higher than 1 atm.

5. The method as claimed in Claim 1 or 2, wherein Step (b) comprises extracting the Chinese herb raw material through water decoction.

6. The method as claimed in any of the preceding claims, wherein the extract in Step (b) comprises saccharides, and glycosides.

7. The method as claimed in any of the preceding claims, further comprising a step of collecting volatile oils in a separate bin after Step (b), and then the volatile oils are added after the granulating process of Step (d).

8. The method as claimed in any of the preceding claims, wherein Step (c) comprises filtering the concentrate.

9. The method as claimed in any of the preceding claims, wherein Step (c) comprises heating the concentrate.

10. The method as claimed in any of the preceding claims, wherein the capsule protective agent is gelatinized starch (alkaline-treated starch), gellan gum, curdlan, alginic acid, acacia, hydroxypropyl methylcellulose (propylene glycol ether of methycellulose), hydroxypropyl cellulose, carrageenan, xanthan gum, maltodextrin or cyclodextrin or a mixture thereof.

11. The method as claimed in any of the preceding claims, further comprising a step of adding a capsule protective agent to the concentrate and mixing the capsule protective agent with the concentrate, after Step (c).

12. The method as claimed in any of the preceding claims, wherein the decomposed product of starch in Step (d) is maltodextrin or cyclodextrin or a mixture thereof.

13. A method for manufacturing extracted, evaporated, granulated botanical herb products such as concentrated Chinese herbs, comprising:
(a) providing a Chinese herb raw material;
(b) obtaining an extract from the Chinese herb raw material;
(c) removing water from the extract, to get a concentrate:
(d) adding a first capsule protective agent to the concentrate and mixing the first capsule protective agent with the concentrate; and
(e) adding an excipient, starch, or a decomposed product of starch to the concentrate, and then granulating and drying, to get a Chinese herbal preparation.

14. The method as claimed in Claim 13, further comprising a step of pre-processing the Chinese herb raw material after Step (a).

15. The method as claimed in Claim 13 or 14, wherein Step (b) comprises extracting the Chinese herb raw material by supercritical carbon dioxide.

16. The method as claimed in Claim 15, wherein Step (b) is performed at a pressure higher than 1 atm.

17. The method as claimed in Claim 13 or 14, wherein Step (b) comprises extracting the Chinese herb raw material through water decoction.

18. The method as claimed in any of claims 13 to 17, wherein the extract in Step (b) comprises saccharides and glycosides.

19. The method as claimed in any of claims 13 to 18, further comprising a step of collecting volatile oils in a separate bin after Step (b), and then the volatile oils are added after the granulating process of Step (e).

20. The method as claimed in any of claims 13 to 19, wherein Step (c) comprises filtering the concentrate.

21. The method as claimed in any of claims 13 to 20, wherein Step (c) comprises heating the concentrate.

22. The method as claimed in any of claims 13 to 21, wherein the first capsule protective agent in Step (d) is selected from a group consisting of gelatinized starch (alkaline-treated starch), gellan gum, curdlan, alginic acid, acacia, hydroxypropyl methylcellulose (propylene glycol ether of methycellulose), hydroxypropyl cellulose, carrageenan, xanthan gum, maltodextrin, cyclodextrin, and a mixture thereof.

23. The method as claimed in any of claims 13 to 22, wherein the excipient in Step (e) comprises a second capsule protective agent of 1-99 wt% with respect to the excipient and a decomposed product of starch of 1-99 wt% with respect to the excipient.

24. The method as claimed in Claim 23, wherein the second capsule protective agent is selected from a group consisting of gelatinized starch (alkaline-treated starch), gellan gum, curdlan, alginic acid, acacia, hydroxypropyl methylcellulose (propylene glycol ether of methycellulose), hydroxypropyl cellulose, carrageenan, xanthan gum, maltodextrin, cyclodextrin, and a mixture thereof.

25. The method as claimed in any of claims 13 to 24, wherein the decomposed product of starch in Step (e) is maltodextrin or cyclodextrin, or a mixture thereof.

26. An excipient used in a method for manufacturing concentrated Chinese herbs or for use in a method for manufacturing concentrated Chinese herbs, comprising a capsule protective agent of 1-99 wt% with respect to the excipient and a decomposed product of starch of 1-99 wt% with respect to the excipient.

27. The excipient as claimed in Claim 26, wherein the capsule protective agent is gelatinized starch (alkaline-treated starch), gellan gum, curdlan, alginic acid, acacia, hydroxypropyl methylcellulose (propylene glycol ether of methycellulose), hydroxypropyl cellulose, carrageenan, xanthan gum, maltodextrin or cyclodextrin or a mixture thereof.

28. The excipient as claimed in Claim 26 or 27, wherein the decomposed product of starch is maltodextrin or cyclodextrin or a mixture thereof.
